Europäisches Patentamt

European Patent Office

Office européen des brevets

(19)

(11) Veröffentlichungsnummer: **0 299 354 B1**

(12) **EUROPÄISCHE PATENTSCHRIFT**

(45) Veröffentlichungstag der Patentschrift: **06.04.94**

(21) Anmeldenummer: **88110842.7**

(22) Anmeldetag: **07.07.88**

(51) Int. Cl.⁵: **C12P 21/00,** A61K 39/395, C12N 15/00, G01N 33/577, A61K 47/00

(54) **Monoklonale Antikörper gegen E7-Protein des Humanen Papillomvirus Typ 16, Verfahren zu ihrer Herstellung sowie ihre Verwendung.**

(30) Priorität: **11.07.87 DE 3722967**

(43) Veröffentlichungstag der Anmeldung:
**18.01.89 Patentblatt 89/03**

(45) Bekanntmachung des Hinweises auf die Patenterteilung:
**06.04.94 Patentblatt 94/14**

(84) Benannte Vertragsstaaten:
**AT BE CH DE ES FR GB IT LI LU NL SE**

(56) Entgegenhaltungen:
**EP-A- 0 256 321**
**EP-A- 0 257 754**

**PROC. NATL. ACAD. SCI. USA, Band 83, Juli 1986, Seiten 4680-4684; D. SMOTKIN et al.**

**JOURNAL OF GEN. VIROL, Band 68, Nr. 11, 19. November 1987, Seiten 2933-2938,SGM, GB; T. OLTERSDORF et al.**

(73) Patentinhaber: **BEHRINGWERKE Aktiengesellschaft**
**Postfach 1140**
**D-35001 Marburg(DE)**

(72) Erfinder: **Oltersdorf, Tilman, Dr.**
**St.-Anna-Gasse 13**
**D-6900 Heidelberg(DE)**
Erfinder: **Röwekamp, Walter, Dr.**
**Emmertsgrundpassage 11**
**D-6900 Heidelberg(DE)**
Erfinder: **Seedorf, Klaus, Dr.**
**15A Woodlawn Ave**
**San Francisco, CA(US)**
Erfinder: **Gissmann, Lutz, Prof. Dr.**
**Alte Bruchsaler Strasse 25**
**D-6908 Wiesloch(DE)**

EP 0 299 354 B1

**Beschreibung**

Humane Papillomviren (HPV) wurden im Zusammenhang mit benignen (Warzen; Kondylome im Genitalbereich) und malignen (Karzinome der Haut und der Genitalschleimhaut) epithelialen Neoplasmen aufgefunden. Da Papillomviren sich nicht in Kultur vermehren lassen, setzt die Gewinnung nennenswerter Mengen viraler Proteine gentechnische Verfahren voraus. Diese Methodik wurde kürzlich angewendet, um polyklonale Antikörper gegen einige HPV-kodierte Proteine herzustellen. Mit diesen polyklonalen Antikörpern gelang wiederum der Nachweis einiger viraler Proteine in HPV-positiven Zellinien, die von Zervixkarzinomen abgeleitet waren (Seedorf et al. (1987) EMBO J 6; 139-144, D. Smotkin und F.O. Wettstein (1986) Proc. Nat. Acad. Sci. USA 83, 4680-4684 und D. Smotkin und F.O. Wettstein (1987), J. Virol. 61, 1686-1689).

Die vorliegende Erfindung betrifft monoklonale Antikörper (MAK), die gegen das 15 kd große E7-Protein von Humanem Papillomvirus 16 (HPV 16 E7) gerichtet sind. Diese Antikörper können als Diagnostikum, Wirkstoff oder Wirkstoffträger verwendet werden. 5 Hybridome konnten gewonnen werden, deren sezernierte MAK (E7II - E7VI) charakterisiert wurden. Alle MAK reagierten positiv in einem "Enzyme linked immunosorbent Assay (ELISA)", an dessen Festphase ein MS-2/HPV16 E7 Fusionsprotein adsorbiert war (s.u.). Im korrespondierenden Western Blot konnte für E7III keine Reaktion nachgewiesen werden. In HPV 16 haltigen Zellinien war E7 Protein im Western Blot mit E7II, E7IV, E7V und E7VI nachweisbar, in der Immunpräzipitation mit E7II und E7IV, wobei E7III und E7V nicht getestet wurden (Tab. 1). In kompetitiven Bindungsstudien zeigten E7II, E7IV, E7V und E7VI stark inhibierende Eigenschaften, während E7III schwächer aber signifikant inhibierte. Eine Mischung von E7II, V, VI präzipitierte HPV16 E7 aus einem in vitro Translationsansatz - von HPV-16-E7-spezifischer RNA, was ebenfalls die Spezifität dieser MAK beweist. E7III ist kreuzreagierend mit HPV18 E7. Der Nachweis von HPV16 E7 gewinnt zusätzlich Bedeutung, da E7 das höchst-exprimierte Protein in HPV16 oder HPV18 enthaltenden Zellinien ist und E7 eine Rolle bei der Aufrechterhaltung des transformierten Phänotyps spielen könnte. Im folgenden ist die weitere Ausgestaltung der Erfindung beschrieben.

**1. a Expression des zur Immunisierung verwendeten MS-2/HPV16 E7 Fusionsproteins**
Plasmidkonstruktionen sowie Isolierung und Reinigung exprimierter HPV16 Proteine sind in Seedorf et al. (1987), EMBO J. 6, 139-144 beschrieben. Das nachfolgend verwendete HPV16 E7 Protein besitzt nicht die ersten 8 Aminosäuren. Es ist mit einem N-terminalen "Leader-Peptid" fusioniert, das aus 100 Aminosäuren der MS-2 Polymerase und 9 Aminosäuren aus Polylinker-Sequenzen besteht und wurde in einer Reinheit von etwa 70 % eingesetzt.

**1. b Enzyme linked immunosorbent Assay (ELISA)-Durchführung**
ELISA-Testplatten (Nunc, Dänemark) wurden über Nacht bei Raumtemperatur mit 0.2-0.5 $\mu$g pro Vertiefung des gereinigten MS-2, MS-2/HPV16 E7 oder MS-2/HPV18 E7 Proteins in 100 $\mu$l 7M Harnstoff inkubiert (12 Stunden, Raumtemperatur). Nach zweimaligem Waschen mit phosphatgepufferter Salzlösung (PBS) wurden die Platten eine Stunde bei 37°C mit 2 %iger Rinderserumalbuminlösung in PBS abgesättigt und waren somit nach 3x Waschen in PBS fertig zum Einsatz. Je 40 $\mu$l zu testende Hybridomzellüberstände pro Vertiefung wurden eine Stunde bei Raumtemperatur inkubiert, nicht gebundene Bestandteile durch 4x Waschen mit PBS, das 0.05 % Tween 20, (PBS/Tween) enthielt, entfernt und nochmals eine Stunde mit durch Meerettichperoxidase konjugierten Ziege-anti-Maus (IgG, IgM)-Immunglobulin (Dianova, Hamburg) in einer Verdünnung von 1: 3000 inkubiert (1 Stunde, Raumtemperatur). Nach viermaligem Waschen mit PBS/Tween zur Entfernung nicht gebundener Reaktanden wurde gebundene Peroxidase durch Inkubation mit $H_2O_2$/Chromogenlösung (0.01 % $H_2O_2$, 1 mg/ml Orthophenylendiamin in 0.1 M Phosphatpuffer pH 5.5) und Extinktionsmessung bei 492 nm nachgewiesen, nachdem nach 10 Minuten die Enzymreaktion durch Zugabe von 50 $\mu$l 1M $H_2SO_4$ gestoppt war.

**2. Hybridomisolierung**
In an sich bekannter Weise (G. Köhler und C. Milstein (1975), Nature 256, 495-497) wurden etwa 500 Hybridome aus Fusionen von Milzzellen immunisierter Balb/c Mäuse mit Myelomzellen erhalten. Dabei sind NS-1 Myelomzellen (Kearny et al.(1979), J. Immunology 123,4 1548-50) mit Milzzellen aus Balb/c Mäusen fusioniert worden. Diese sechs Wochen alten Balb/c Mäuse waren vorher im 2 Wochen Intervall zweimal subkutan, darauf einmal drei Tage von Fusion intraperitoneal mit jeweils 10-20 $\mu$g des unter 1. beschriebenen MS-2/HPV16 E7 Fusionsproteins immunisiert worden.

Hybridome, die Antikörper gegen HPV 16 E7 sezernieren ließen sich durch Paralleltestung in zwei "Enzyme linked immunosorbent Assays (ELISA)" nachweisen. Dabei war an der Festphase des einen ELISA MS-2/HPV16 E7 Fusionsprotein adsorbiert, an der Festphase des anderen nur MS-2 Protein. Gebundene Antikörper von Hybridomüberständen wurden durch Inkubation mit Ziege-Anti-Maus Antikörpern nachgewiesen, die mit Meerrettichperoxidase konjugiert waren. Bei den oben genannten zirka 500

2

Hybridomen waren etwa 150 Klone positiv beim Screening mit dem "MS-2 ELISA", 10 Klone waren zusätzlich positiv mit dem "MS-2/HPV16 E7 ELISA". Von diesen 10 Klonen waren nach zwei Klonierungsrunden zur Erzeugung von Einzelklonen auf einem "Feeder-Layer" von peritonealen Mausmakrophagen 6 stabil (Tab. 1).

### 3. Charakterisierung HPV16 E7 - spezifischer MAK

Im "Western Blot", durchgeführt im wesentlichen nach Towbin et al. (1979), Proc. Natl. Acad. Sci. U.S.A. 76, 4350-4354, reagierten die sezernierten MAK von 4 Hybridomen (E7 II, E7 IV, E7 V, E7 VI) mit dem MS-2/HPV16 E7 Fusionsprotein und nicht mit MS-2 Protein allein. Zur Testung der Typspezifität wurden die genannten 4 Hybridome mit einem ELISA analog wie unter 2. beschrieben untersucht, wobei aber das MS-2/HPV18 E7 an der Festphase adsorbiert war. MAK des Hybridoms E7 IV reagierten positiv, jedoch betrugen die Extinktionswerte in diesem ELISA weniger als 50 % der Werte mit dem entsprechenden MS-2/HPV16 E7-ELISA.

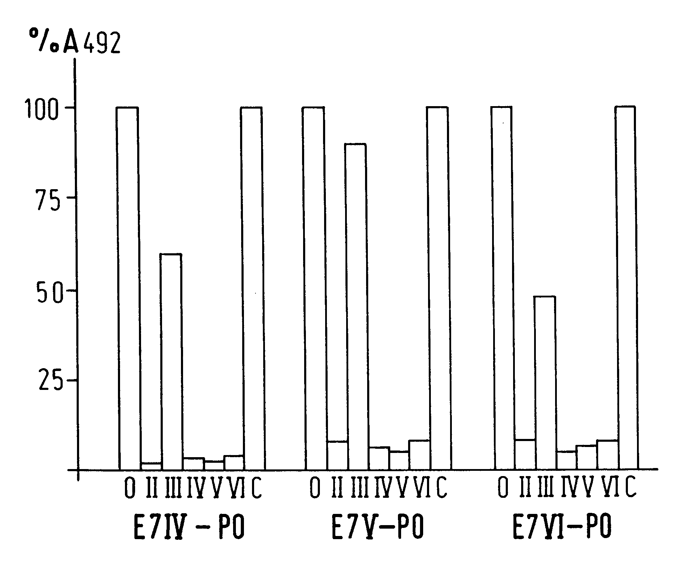

Kompetitive Bindungsteste zur weiteren Analyse der MAK wurden mit einem ELISA wie unter 1.b beschrieben durchgeführt (MS-2/HPV-16 E7 Protein an der Festphase). Dabei wurden E7 IV, E7 V und E7 VI MAK als Meerettich-Peroxidasekonjugate (Ishikawa et al. (1983), J. Immunoassay 4, 209-327) mit je 5 $\mu$g/ml eingesetzt, während die unkonjugierten MAK E7 II, III, IV, V, VI in seriellen Verdünnungen von 50 $\mu$g/ml (= 100 % Inhibition des homologen MAK-Konjugats) zur Anwendung gelangten. In diesen Versuchen (Fig. 1) inhibieren alle MAK sich gegenseitig, die im Western Blot mit HPV16 E7 reagierten (MAK E II, E IV, E V, E VI). Der MAK E7 III, der nur im HPV16 E7-spezifischen ELISA reagierte, inhibiert die anderen MAK deutlich weniger aber immer noch signifikant gegenüber einem Kontrollantikörper anderer Spezifität (Fig. 1, Reihen C). In Fig. 1 ist an der Ordinate die relative Extinktion (% A 492) gegenüber den verschiedenen kompetitiven Bindungstestkombinationen auf der Abszisse aufgetragen, wobei O keine Zugabe von unmarkiertem MAK bedeutet (entsprechend 100 % A 492). Diese Ergebnisse legen nahe, daß die MAK E7 II, E7 IV, E7 V und E7 VI mit topologisch sehr ähnlichen aber nicht identischen Epitopen reagieren, wie auch die Kreuzreaktion von E7 IV mit HPV18 E7 zeigt. Wahrscheinlich reagiert E7 III mit einem nah benachbarten Epitop.

### 4. Nachweis von HPV16 E7 durch MAK in Zellinien, die HPV16 enthalten.

#### a) Western Blot

Western Blots wurden durchgeführt wie bekannt (Towbin et al. a.a.O.), nachdem die Zellextrakte der CaSKi Zellinie (R.A. Patillio (1977), Science 196, 1456-58)bzw. der SiHa Zellinie (Friedl, F. et al. (1979), Proc. Soc. Experiment. Biol. and Med. 135, 543-545) vorher in 10 % SDS Polyacrylamidgelelektrophorese im wesentlichen wie beschrieben (Laemmli, V.K. (1970), Nature 227, 680-682) aufgetrennt waren. Mit E7 II, E7 IV, E7 V und E7 VI konnte das 15kd große HPV16 E7 Protein in beiden Zellinien nachgewiesen werden.

#### b) Immunpräzipitation

Immunpräzipitationen wurden nach N. Koch und G.J. Hämmerling (1982), J. Immunol. 128, 1155-1157 durchgeführt. E7 II und E7 IV präzipitierten HPV16 E7 aus CaSKi-Zellen, während bei Kontrollzellen ohne HPV keine spezifischen Präzipitate erhalten wurden.

In Tabelle 1 sind die Eigenschaften der gewonnenen MAK (E7 I - E7 VI) zusammengefaßt.

| M A K Antikörper Isotyp | | Reaktivität mit | | | |
|---|---|---|---|---|---|
| | | MS-2/HPV16 E7 in | | E7 Protein (CaSKi oder SiHa Zellen) in | |
| | | ELISA | Western Blot | Western Blot | Immunoprezipitation |
| E7 I | IgG 1 | + | + | unspezifisch | |
| E7 II | IgG 2a | + | + | + | + |
| E7 III | IgG 1 | + | − | − | nd* |
| E7 IV | IgG 2a | + | + | + | + |
| E7 V | IgG 1 | + | + | + | nd* |
| E7 VI | IgG 1 | + | + | + | − |

Tab. 1: Isotypen der HPV16 E7 spezifischen MAK undihre Reaktivität.

*) nicht durchgeführt.

Die Hinterlegungsnummern der European Collection of Animal Cell Cultures (ECACC) für die o.g. MAK, die in Form der Hybridome hinterlegt wurden, sind wie folgt (E7 II - E7 VI): 88062429, 88062430, 88062431, 88062432, 88062433.

**Patentansprüche**
**Patentansprüche für folgende Vertragsstaaten : AT, BE, CH, DE, FR, GB, IT, LI, LU, NL, SE**

1. Hybridome mit der Hinterlegungsnummer ECACC 88 06 24 29, ECACC 88 06 24 30, ECACC 88 06 24 31, ECACC 88 06 24 32 und ECACC 88 06 24 33.

2. Monoklonale Antikörper E7II, E7III, E7IV, E7V und E7VI, die von den Hybridomen nach Anspruch 1 sezerniert werden.

3. Verfahren zur Herstellung von monoklonalen Antikörpern nach Anspruch 2, dadurch gekennzeichnet, daß Hybridome gemäß Anspruch 1 kultiviert und die sezernierten Antikörper isoliert werden.

4. Verfahren zum in vitro Nachweis von humanen Papillomaviren, dadurch gekennzeichet, daß einer oder mehrere Antikörper nach Anspruch 2 eingesetzt werden.

5. Verwendung eines monokonalen Antikörpers nach Anspruch 2 in einem Diagnostikum.

6. Verwendung eines monoklonalen Antikörpers nach Anspruch 2 für einen pharmazeutischen Wirkstoff.

7. Verwendung eines monoklonalen Antikörpers nach Anspruch 2 zur Herstellung eines Arzneimittels zur therapeutischen Anwendung.

8. Verwendung eines monoklonalen Antikörpers nach Anspruch 5 oder 7, dadurch gekennzeichnet, daß der Antikörper markiert ist.

9. Verwendung eines monoklonalen Antikörpers nach Anspruch 2 zur Herstellung eines Diagnostikums für die Analyse von Geweben und Körperflüssigkeiten und für die Radioimmunszintigraphie.

EP 0 299 354 B1

10. Verwendung eines monoklonalen Antikörpers nach Anspruch 2 zur Herstellung eines Therapeutikums für die Radioimmuntherapie oder für die Chemoimmuntherapie.

**Patentansprüche für folgenden Vertragsstaat : ES**

1. Verfahren zur Herstellung von monoklonalen Antikörpern E7II, E7III, E7V und E7VI, dadurch gekennzeichnet, daß Hybridome mit der Hinterlegunsnummer ECACC 88 06 24 29, ECACC 88 06 24 30, ECACC 88 06 24 31, ECACC 88 06 24 32 und ECACC 88 06 24 33 kultiviert und die sezernierten Antikörper isoliert werden.

2. Verfahren zum in vitro Nachweis von humanen Papillomaviren, dadurch gekennzeichnet, daß einer oder mehrere der nach Anspruch 1 hergestellten monoklonalen Antikörper eingesetzt wird.

3. Verwendung eines monoklonalen Antikörpers hergestellt nach einem Verfahren gemäß Anspruch 1 in einem Diagnostikum.

4. Verwendung eines monoklonalen Antikörpers hergestellt nach einem Verfahren gemäß Anspruch 1 für einen pharmazeutischen Wirkstoff.

5. Verwendung eines monoklonalen Antikörpers hergestellt nach einem Verfahren gemäß Anspruch 1 zur Herstellung eines Arzneimittels zur therapeutischen Anwendung.

6. Verwendung eines monoklonalen Antikörpers nach Anspruch 3 oder 5, dadurch gekennzeichnet, daß der Antikörper markiert ist.

7. Verwendung eines monoklonalen Antikörpers hergestellt nach einem Verfahren gemäß Anspruch 1 zur Herstellung eines Diagnostikums für die Analyse von Geweben und Körperflüssigkeiten und für die Radioimmunszintigraphie.

8. Verwendung eines monoklonalen Antikörpers hergestellt nach einem Verfahren gemäß Anspruch 1 zur Herstellung eines Therapeutikums für die Radioimmuntherapie oder für die Chemoimmuntherapie.

**Claims**

**Claims for the following Contracting States : AT, BE, CH, DE, FR, GB, IT, LI, LU, NL, SE**

1. Hybridomas having the deposition number ECACC 88 06 24 29, ECACC 88 06 24 30, ECACC 88 06 24 31, ECACC 88 06 24 32 and ECACC 88 06 24 33.

2. Monoclonal antibodies E7II, E7III, E7IV, E7V and E7VI, which are secreted by the hybridomas as claimed in claim 1.

3. A process for producing monoclonal antibodies as claimed in claim 2, which comprises hybridomas as claimed in claim 1 being cultivated and the secreted antibodies being isolated.

4. A method for the in vitro detection of human papillomaviruses, wherein one or more antibodies as claimed in claim 2 are used.

5. The use of a monoclonal antibody as claimed in claim 2 in a diagnostic aid.

6. The use of a monoclonal antibody as claimed in claim 2 for a pharmaceutical active compound.

7. The use of a monoclonal antibody as claimed in claim 2 for producing a drug for therapeutic use.

8. The use of a monoclonal antibody as claimed in claim 5 or 7, wherein the antibody is labeled.

9. The use of a monoclonal antibody as claimed in claim 2 for producing a diagnostic aid for the analysis of tissues and body fluids and for radioimmunoscintigraphy.

5

EP 0 299 354 B1

**10.** The use of a monoclonal antibody as claimed in claim 2 for producing a therapeutic for radioimmunotherapy or for chemoimmunotherapy.

**Claims for the following Contracting State : ES**

**1.** A process for producing monoclonal antibodies E7II, E7III, E7IV, E7V and E7VI, which comprises hybridomas having the deposition number ECACC 88 06 24 29, ECACC 88 06 24 30, ECACC 88 06 24 31, ECACC 88 06 24 32 and ECACC 88 06 24 33 being cultivated and the secreted antibodies being isolated.

**2.** A method for the in vitro detection of human papillomaviruses, wherein one or more monoclonal antibodies produced as claimed in claim 1 are used.

**3.** The use of a monoclonal antibody produced by a process as claimed in claim 1 in a diagnostic aid.

**4.** The use of a monoclonal antibody produced by a process as claimed in claim 1 for a pharmaceutical active compound.

**5.** The use of a monoclonal antibody produced by a process as claimed in claim 1 for producing a drug for therapeutic use.

**6.** The use of a monoclonal antibody as claimed in claim 3 or 5, wherein the antibody is labeled.

**7.** The use of a monoclonal antibody produced by a process as claimed in claim 1 for producing a diagnostic aid for the analysis of tissues and body fluids and for radioimmunoscintigraphy.

**8.** The use of a monoclonal antibody produced by a process as claimed in claim 1 for producing a therapeutic for radioimmunotherapy or for chemoimmunotherapy.

**Revendications**
**Revendications pour les Etats contractants suivants : AT, BE, CH, DE, FR, GB, IT, LI, LU, NL, SE**

**1.** Hybridomes portant les numéros de dépôt ECACC 88 06 24 29, ECACC 88 06 24 30, ECACC 88 06 24 31, ECACC 88 06 24 32 et ECACC 88 06 24 33.

**2.** Anticorps monoclonaux E7II, E7III, E7IV, E7V et E7VI, qui sont sécrétés par les hybridomes selon la revendication 1.

**3.** Procédé pour la production d'anticorps monoclonaux selon la revendication 2, caractérisé en ce que l'on cultive des hybridomes selon la revendication 1 et on isole les anticorps sécrétés.

**4.** Procédé pour la détection in vitro de papillomavirus humains, caractérisé en ce que l'on utilise un ou plusieurs anticorps selon la revendication 2.

**5.** Utilisation d'un anticorps monoclonal selon la revendication 2, dans un agent de diagnostic.

**6.** Utilisation d'un anticorps monoclonal selon la revendication 2, pour une substance active pharmaceutique.

**7.** Utilisation d'un anticorps monoclonal selon la revendication 2, pour la fabrication d'un médicament à application thérapeutique.

**8.** Utilisation d'un anticorps monoclonal selon la revendication 5 ou 7, caractérisé en ce que l'anticorps est marqué.

**9.** Utilisation d'un anticorps monoclonal selon la revendication 2, pour la préparation d'un agent de diagnostic pour l'analyse de tissus et de liquides corporels et pour la radio-immunoscintigraphie.

6

**10.** Utilisation d'un anticorps monoclonal selon la revendication 2, pour la préparation d'un agent thérapeutique pour la radio-immunothérapie ou pour la chimio-immunothérapie.

**Revendications pour l'Etat contractant suivant : ES**

**1.** Procédé pour la production d'anticorps monoclonaux E7II, E7III, E7IV, E7V et E7VI, caractérisé en ce que l'on cultive des hybridomes portant les numéros de dépôt ECACC 88 06 24 29, ECACC 88 06 24 30, ECACC 88 06 24 31, ECACC 88 06 24 32 et ECACC 88 06 24 33 et on isole les anticorps sécrétés.

**2.** Procédé pour la détection in vitro de papillomavirus humains, caractérisé en ce que l'on utilise un ou plusieurs des anticorps monoclonaux produits selon la revendication 1.

**3.** Utilisation d'un anticorps monoclonal, préparé selon un procédé conforme à la revendication 1, dans un agent de diagnostic.

**4.** Utilisation d'un anticorps monoclonal, produit selon un procédé conforme à la revendication 1, pour une substance active pharmaceutique.

**5.** Utilisation d'un anticorps monoclonal, produit selon un procédé conforme à la revendication 1, pour la fabrication d'un médicament à application thérapeutique.

**6.** Utilisation d'un anticorps monoclonal selon la revendication 3 ou 5, caractérisé en ce que l'anticorps est marqué.

**7.** Utilisation d'un anticorps monoclonal, produit selon un procédé conforme à la revendication 1, pour la préparation d'un agent de diagnostic pour l'analyse de tissus et de liquides corporels et pour la radio-immunoscintigraphie.

**8.** Utilisation d'un anticorps monoclonal, produit selon un procédé conforme à la revendication 1, pour la préparation d'un agent thérapeutique pour la radio-immunothérapie ou pour la chimio-immunothérapie.